# FASCICULE DE BREVET EUROPEEN

(11) **EP 3 324 881 B1**
(45) Date de publication et mention de la délivrance du brevet: **08.01.2025**
(21) Numéro de dépôt: 16727752.4
(22) Date de dépôt: 10.05.2016
(51) Int. Cl.: A61F 2/00

(54) **PROTHÈSES DE RÉPARATION DE HERNIES**
PROTHESE FÜR HERNIENREPARATUR
HERNIA REPAIR PROSTHESES

(30) Priorité: 20.07.2015 FR 1556867
(43) Date de publication de la demande: 30.05.2018
(73) Titulaire: THT Bio-Science, 34220 Verreries de Moussans (FR)
(72) Inventeur: PELISSIER, Edouard, 25870 Geneuille (FR); LARGENTON, Claude, 50000 Saint Lo (FR); NGO, Philippe, 75116 Paris (FR)
(74) Mandataire: Demulsant, Xavier
(86) Numéro de dépôt international: PCT/FR2016/051093
(87) Numéro de publication internationale: WO 2017/013320

(56) Documents cités:
- EP-A1- 2 395 941
- WO-A1-2013/122700
- WO-A1-2014/167131
- WO-A1-2015/011417
- FR-A1- 2 924 330
- US-A1- 2014 350 580

## Description

La présente invention concerne les prothèses pour la réparation de hernies, et tout particulièrement les prothèses adaptées à la réparation de hernies inguinales.

Les hernies sont définies comme l'issue spontanée, temporaire ou permanente, d'un sac péritonéal contenant une partie ou la totalité d'un ou plusieurs viscères ou du grand épiploon hors des limites de la cavité les contenant normalement, au travers d'une zone de faiblesse anatomiquement prévisible et parfois favorisée par une prédisposition congénitale ou acquise.

Les hernies peuvent entraîner des douleurs, une gêne à la marche, des troubles du transit, et dégradent l'image corporelle.

La complication la plus grave des hernies est l'étranglement herniaire : son traitement est une urgence chirurgicale. L'étranglement de l'intestin grêle, le plus fréquent et le plus grave, est responsable d'une occlusion aigüe, son évolution sans traitement se fait vers la nécrose de l'intestin dans le sac herniaire et engage le pronostic vital.

Le document WO2015/011417 décrit une prothèse intrapéritonéale, comprenant une face adhérente au péritoine et une face non adhérente dans la cavité abdominale, au contact de l'intestin, pour le traitement des hernies ventrales, et vise à éviter l'adhérence au bord périphérique de la prothèse.

Le document FR 2924330 décrit un implant spécifiquement conçu pour la prévention ou le traitement de hernie à proximité d'une stomie de paroi abdominale (iléostomie, colostomie).

La hernie de l'aine est une affection fréquente, la cure chirurgicale de la hernie de l'aine étant l'intervention la plus pratiquée en chirurgie abdominale :
- 160 000 cas par an en France *(*Oberlin et al, Le traitement des hernies de l'aine en 1998 : un exemple de la disparité des pratiques, Drees, Etudes et Résultats n°92, 2000*),* l'incidence globale étant de 272 interventions annuelles pour 100 000 habitants;
- 800 000 cas aux Etats-Unis *(*Aquina et al, The pitfalls of inguinal herniorrhaphy, surgeon volume matters, Surgery, 2015*);*
- 80 000 cas en Grande Bretagne *(*Bhattacharjee, Surgical options in inguinal hernia: which is the best, Indian J Surg, 68, pp. 191-197, 2006*).*

Le traitement chirurgical des hernies de l'aine concerne surtout les hommes, avec plus de huit interventions sur dix.

Selon l'orifice par lequel la hernie est extériorisée, on distingue les hernies inguinales et les hernies fémorales. Les hernies fémorales ou crurales ne représentent qu'environ 3% des hernies de l'aine.

Diverses classifications des hernies ont été proposées *(*Miserez et al, The European hernia society groin hernia classification, Hernia, 2007*)* et les hernies inguinales se répartissent en trois types principaux, selon le siège de la déhiscence : hernies directes, hernies indirectes et hernies mixtes. Les hernies inguinales sont définies par le passage à travers le fascia transversalis, qui constitue la paroi postérieure du canal inguinal, d'un diverticule péritonéal, le sac herniaire, contenant ou non des viscères. Ce sac est précédé par un lipome pré-herniaire, plus ou moins volumineux, situé le plus souvent en dehors du cordon spermatique.

Chez l'enfant, la fermeture de l'orifice herniaire se fait toujours par suture des tissus.

Chez l'adulte, les méthodes de raphie, par suture (technique de Bassini développée en 1887, technique de Shouldice développée au début des années 50) ont prévalu jusque dans les années 1990, avec un pourcentage de récidive élevé, de l'ordre de 8%, et ont été progressivement remplacées par des procédés comportant la mise en place d'une prothèse *(*Pélissier, Etat actuel du traitement de la hernie inguinale, e-mémoire de l'académie de chirurgie 8(2), pp. 31-33, 2009*).*

Il existe aujourd'hui une très grande variété d'implants pour le traitement des hernies abdominales *(*Zogbi, The use of biomaterials to treat abdominal hernias, in Biomaterials applications for nanomedecine, ISBN 978-953-307-661-4, 2011*)* et plus particulièrement des hernies de l'aine *(*Bilsel et al, The search for idéal hernia repair, mesh materials and types, International Journal of Surgery 10, pp. 317-321, 2012*).* Les prothèses sont conventionnellement des pièces de treillis synthétique, le plus souvent en fils de polypropylène ou polyéthylène. Ces filets provoquent une réaction fibro-scléreuse de l'organisme, responsable de leur incorporation dans les tissus, qui est le gage de la solidité de la réparation. En contrepartie, les adhérences peuvent provoquer des effets secondaires, lorsqu'elles se font avec des organes.

Les réparations prothétiques des hernies de l'aine ont fait diminuer le pourcentage de récidive, qui est maintenant de l'ordre de 1 à 2%, de sorte qu'actuellement la préoccupation principale des chirurgiens n'est plus la récidive, mais le risque de douleur chronique et son retentissement sur la qualité de vie *(*Shouldice, International Journal of Clinical Medicine, 5, pp. 737-740, 2014*).* L'incidence de la douleur chronique est estimée en moyenne à 10-12% et elle retentit sur l'activité, y compris professionnelle, dans 0.5 à 6% des cas.

Il existe deux grands types de techniques opératoires pour la mise en place de la prothèse : les techniques de type Lichtenstein et les techniques pré-péritonéales.

Dans les techniques de type Lichtenstein, la hernie est abordée par incision directe (laparotomie) au niveau de l'aine, et la prothèse est fixée à la surface du plan musculaire. Ces techniques ont un faible pourcentage de récidive (de l'ordre de 1 à 2%), mais exposent à des douleurs post-opératoires, et surtout des douleurs chroniques, ainsi que des troubles de la sensibilité cutanée. Ces complications sont liées à un risque de lésion des nerfs sensitifs de la région inguinale. Ce risque est lié à la dissection étendue, au risque de prendre les nerfs dans la suture qui est nécessaire pour fixer la prothèse et au risque d'englobement des nerfs dans la gangue fibro-scléreuse provoquée par la prothèse.

Dans les techniques pré-péritonéales, la pose de la prothèse à la face profonde du plan musculaire, dans l'espace compris entre paroi musculaire et péritoine (espace prépéritonéal) peut se faire :
- par voie endoscopique (laparoscopie, coeliochirurgie), par
   ∘ voie extrapéritonéale, dite « Total Extraperitoneal hernioplasty » TEP,
   ∘ ou voie intrapéritonéale dite « Transabdominal Preperitoneal hernioplasty » TAPP *(*Guidelines for TAPP and TEP treatment of inguinal hernia, IEHS, Surg Endosc 25, pp. 2773-2843, 2011*),*
- ou par incision directe Kugel, TIPP (Transinguinal Preperitoneal Patch), TREPP (Trans Rectus sheath Extra-Peritoneal Procedure) et ONSTEP *(*Anderson et al, The Initial Expérience of Introducing the Onstep Technique for Inguinal Hernia Repair in a General Surgical Department, Scandinavian Journal of Surgery, 2014*).*

Les techniques pré-péritonéales par voie endoscopique ou ouverte ont notamment pour avantage, par rapport aux techniques de type Lichtenstein, de réduire la durée d'arrêt de travail, à l'issue de laquelle la majorité des patients est capable de reprendre un travail. A titre indicatif, selon la pratique française, pour un travail physique léger, la durée de référence indicative, selon l'assurance maladie, est de 10 jours pour un traitement par célioscopie et de 21 jours pour un traitement par chirurgie à ciel ouvert.

Les techniques pré-péritonéales par voie laparoscopique ou ouverte ont également pour avantage de réduire les temps d'hospitalisation.

Ces techniques sont aussi efficaces que la technique de Lichtenstein, avec un pourcentage de récidive comparable, et donnent moins de douleur et de troubles de la sensibilité *(*Bobo et al, Meta-analysis of randomized controlled trials comparing Lichtenstein and totally extraperitoneal laparoscopie hernioplasty in treatment of inguinal hernias, Journal of surgical research 192, pp. 409-420, 2014*).* Ceci est dû à l'absence de dissection étendue du canal inguinal, à l'absence de contact de la prothèse avec les nerfs du canal inguinal et au fait que la prothèse ne nécessite que peu ou pas de fixation, étant appliquée contre la paroi, par la pression abdominale. Plusieurs études ont montré que, par rapport aux procédés d'abord direct, la réparation prothétique pré-péritonéale donne :
- moins de douleur postopératoire et permet une reprise plus rapide d'activité ;
- moins de douleur chronique, moins de troubles de la sensibilité ;
- une meilleure qualité de vie.

La prothèse pré-péritonéale entraîne l'adhérence de la prothèse à la vessie et au péritoine, qui provoque des difficultés de dissection lors d'une prostatectomie radicale ultérieure. Ces risques sont mentionnés dans la littérature depuis plus de quinze ans, sans qu'une solution satisfaisante ait été proposée :
- Picozzi et al, Worl J Urol 33, pp. 69-67, 2015*;*
- Spernat et al, Prostate Int 2(1), pp. 8-11, 2014*;*
- Haifler et al, Journal of Endourology, 26(11), pp. 1458-1462, 2012*;*
- Peeters et al, British Journal of Surgery pp, pp. 431-435, 2012*;*
- Neff et al, Urologie Oncology 29, pp. 66-69, 2011*;*
- Do et al, Urology 77(4), pp. 963-967, 2011*;*
- Saint Elie et al, Urology 76(5), pp. 1078-1082, 2010*;*
- Hocaoglu et al, Bju Int 106, pp. 1628-1631, 2010*;*
- Siddiqui et al, Urology 75(5), pp. 1079-1082, 2010*;*
- Lallas et al, JSLS 13, pp. 142-147, 2009*;*
- Tsivian et al, Hernia 13, pp. 523-527, 2009*;*
- Thomas et al, J Am Coll Surg, pp. 371-376, 2009*;*
- Vijan et al, Hernia 12, pp. 415-419, 2008*;*
- Stolzenburg et al, World J Urol 23, pp. 295-299, 2005*;*
- Stolzenburg et al, Adult Urology, pp. 325-331, 2005*;*
- Joseph et al, JSLS 9, pp. 368-369, 2005*;*
- Amid, Hernia 8, pp. 169-170, 2004*;*
- Brown et al, Urology 63(2), pp. 380vii-ix, 2004*;*
- Cooperberg et al, Surgery, pp. 452-453, 2004*;*
- Cook et al, BJU Int 91, pp. 729, 2003*;*
- Borchers et al, Urologia Internationals, 67 pp. 213-215, 2001*;*
- Stoppa et al, Hernia 2, pp. 35-38, 1998*.*

Les chirurgiens urologues se heurtent à des difficultés de dissection pour aborder la prostate, en raison de l'adhérence de la prothèse à la face antérieure de la vessie et à la région prostatique. Par ailleurs, la prostatectomie radicale est habituellement complétée par l'ablation des ganglions lymphatiques drainant la prostate, dans une zone comprise entre les vaisseaux iliaques et le pédicule vasculo-nerveux obturateur (curage ganglionnaire). Or, cette zone est également recouverte par la prothèse et l'adhérence aux vaisseaux entraîne un risque de blessure vasculaire, dont les conséquences peuvent être dramatiques. Pour cette raison, le curage ganglionnaire n'est réalisé que dans la moitié des cas, ce qui peut avoir un impact préjudiciable sur le pronostic du cancer.

En cas de cancer de la vessie chez l'homme, la prostate peut être enlevée en même temps que la vessie. Il a été constaté que la prothèse de traitement de hernie inguinale entraîne des difficultés de dissection, lors d'une cystoprostatectomie radicale, et peut empêcher ou réduire l'extension de la lymphadenectomie *(*Jones, Urology 70(6), pp. 1079-1081, 2007*).*

Réduire l'adhérence de la prothèse à la vessie, à la prostate et à la zone du curage ganglionnaire serait un progrès depuis longtemps attendu, facilitant la prostatectomie radicale et le curage ganglionnaire chez les sujets qui ont bénéficié précédemment de la cure de hernie par prothèse prépéritonéale. Ce progrès est d'autant plus souhaitable que le cancer de la prostate est, en France, selon l'institut de veille sanitaire, le plus fréquent chez l'homme, avec 57 000 nouveaux cas par an (*Binder et al, ISBN 978-2-11-138316-6*, *2012*)*,* le cancer de la prostate étant un des plus fréquents chez l'homme, tous pays confondus.

Un premier objet de l'invention est de proposer des prothèses, pour le traitement des hernies inguinales, facilitant une chirurgie ultérieure, en particulier une prostatectomie radicale, ou une cystoprostatectomie radicale.

Un deuxième objet de l'invention est de proposer des prothèses pour le traitement des hernies inguinales, facilitant une lymphadenectomie ultérieure, lors du traitement d'un cancer de la prostate ou de la vessie.

Un troisième objet de l'invention est de proposer des prothèses pré-péritonéales pour le traitement des hernies inguinales, réduisant les risques d'adhérence entre la prothèse et les viscères.

A ces fins, il est proposé, selon un premier aspect, une prothèse pour la réparation d'une hernie inguinale, destinée à être implantée par voie pré-péritonéale par voie endoscopique ou ouverte, comprenant un textile ajouré en matériau biocompatible, comprenant une première face, dite face pariétale, destinée à être placée en regard des tissus biologiques de la région inguinale, et une deuxième face, opposée à ladite première face, dite face péritonéale, destinée à être placée en regard du péritoine et de la face antérieure de la vessie, la face péritonéale comprenant, sur une partie de sa surface, une première zone pourvue d'un revêtement anti adhérent, la face pariétale comprenant, sur une partie de sa surface, une deuxième zone pourvue d'un revêtement anti adhérent.

Par cette disposition, lors d'une réparation de hernie inguinale, la première zone pourvue d'un revêtement anti adhérent (dite première zone anti adhérente) peut être placée en regard de l'appareil vésico-prostatique et la deuxième zone pourvue d'un revêtement anti adhérent (dite deuxième zone anti adhérente) peut être placée en regard de la zone de curage ganglionnaire. L'étendue de la face pariétale, hors de la deuxième zone anti adhérente, préserve la faculté d'adhésion de la prothèse.

Par « *revêtement anti adhérent »* on désigne ici la présence d'un matériau anti adhérent sur la surface du textile de la prothèse, et/ou un état de surface à faible rugosité.

Le revêtement anti adhérent présent dans la première zone anti adhérente peut être identique ou être différent du revêtement anti adhérent présent dans la deuxième zone anti adhérente. Ainsi, par exemple, un revêtement à base de silicone ou d'hydrogel forme la première zone anti adhérente, la deuxième zone anti adhérente étant définie par la présence, dans cette deuxième zone, d'une très faible rugosité de surface.

Selon diverses mises en oeuvre, le revêtement anti adhérent se présente sous la forme d'un film continu, ou sous la forme de bandes ou de points. Par « *points »* on désigne ici une étendue de dimension réduite, ces points pouvant former un motif régulier, ou être disposés aléatoirement.

La première zone et la deuxième zone anti adhérentes sont disposées sur deux faces opposées de la prothèse. La première zone et la deuxième zone anti adhérente ne sont sensiblement pas disposées à la verticale l'une de l'autre, lorsque la prothèse est vue en plan. Par cette disposition, la présence d'un revêtement anti adhérent n'entraîne pas de surépaisseur importante dans la prothèse, pouvant gêner son introduction dans un trocart.

Selon la présente demande, on entend par « *textile* » tout arrangement ou assemblage de fils, fibres, filaments et/ou multi-filaments, par exemple obtenu par tricotage, tissage, tressage ou encore non tissé, et on entend par « *textile ajouré »* tout textile dont l'arrangement de fils dont il est constitué détermine des jours, alvéoles, pores ou vides dans l'épaisseur du textile, ces jours, alvéoles, pores ou vides pouvant constituer des canaux débouchant de part et d'autre du textile.

Diverses caractéristiques supplémentaires peuvent être prévues, seules ou en combinaison :
- la première zone anti adhérente s'étend sur une surface de l'ordre de 30% à 70% de la surface totale de la face péritonéale ;
- selon la présente invention, la prothèse est de forme rectangulaire, la première zone anti adhérente s'étend sur toute la largeur de la prothèse, depuis un bord latéral de la prothèse ;
- selon la présente invention, la prothèse est de forme rectangulaire, la deuxième zone anti adhérente s'étend sur une largeur comprise entre le tiers et la moitié de la largeur de la prothèse ;
- la première zone et/ou la deuxième zone anti adhérente comprend un revêtement anti adhérent définissant un film continu ;
- le revêtement anti adhérent de la première zone et/ou de la deuxième zone anti adhérente est discontinu et comprend des bandes ou points ;
- le textile ajouré est réalisé à partir de polyéthylène, polypropylène, polytétrafluoroéthylène, polyéthylène téréphtalate, polyfluorure de vinylidène (PVDF) ;
- la première zone et/ou la deuxième zone anti adhérente comprend un film non résorbable ;
- la première zone et/ou la deuxième zone anti adhérente comprend un gel résorbable ;
- le textile comporte une première partie, dite partie supérieure, destinée à être placée en regard de la paroi musculaire antérieure, et du ligament pectiné, et une deuxième partie, dite partie inférieure, destinée à être placée en regard du pubis et des vaisseaux iliaques et spermatiques latéralement, et d'une partie du muscle psoas, la partie supérieure représentant environ entre la moitié et les deux tiers de la surface de la prothèse, le textile comprenant une ligne délimitant une frontière entre la partie supérieure et la partie inférieure ;
- la ligne délimitant la frontière entre la partie supérieure et la partie inférieure de la prothèse comprend une couture ;
- la prothèse est pourvue d'un moyen d'indication de l'orientation de la prothèse ;
- ledit moyen d'indication de l'orientation de la prothèse comprend une zone ayant une couleur différente du reste de la prothèse ;
- le textile étant sous la forme d'un tricot, la zone de couleur différente est obtenue par tricotage d'un fil de couleur différente du ou des fils utilisés pour le tricotage du reste du textile, ou par impression sur le textile.

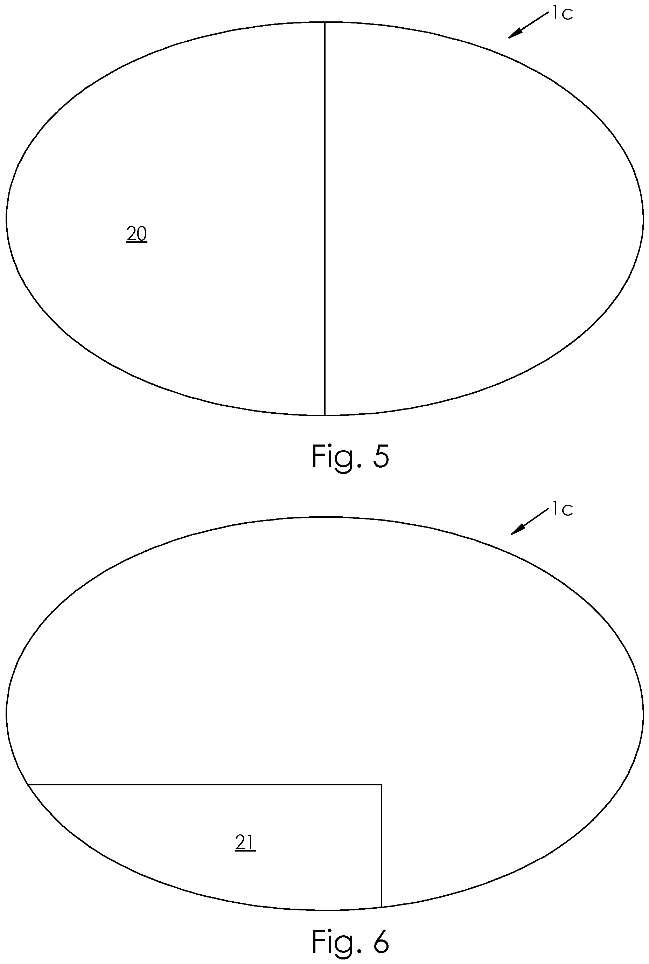

Les exemples illustrés aux figures 5 et 6 ne représentent pas des réalisations de l'invention.
- la figure 1 est une vue en plan d'une prothèse, selon un premier mode de réalisation, la face visible étant la face péritonéale ;
- la figure 2 est une vue en plan de la prothèse de la figure 1, la face visible étant la face pariétale ;
- la figure 3 est une vue en plan d'une prothèse, selon un deuxième mode de réalisation, la face visible étant la face péritonéale ;
- la figure 4 est une vue en plan de la prothèse de la figure 3, la face visible étant la face pariétale ;
- la figure 5 est une vue en plan d'une prothèse, la face visible étant la face péritonéale ;
- la figure 6 est une vue en plan de la prothèse de la figure 5, la face visible étant la face pariétale.

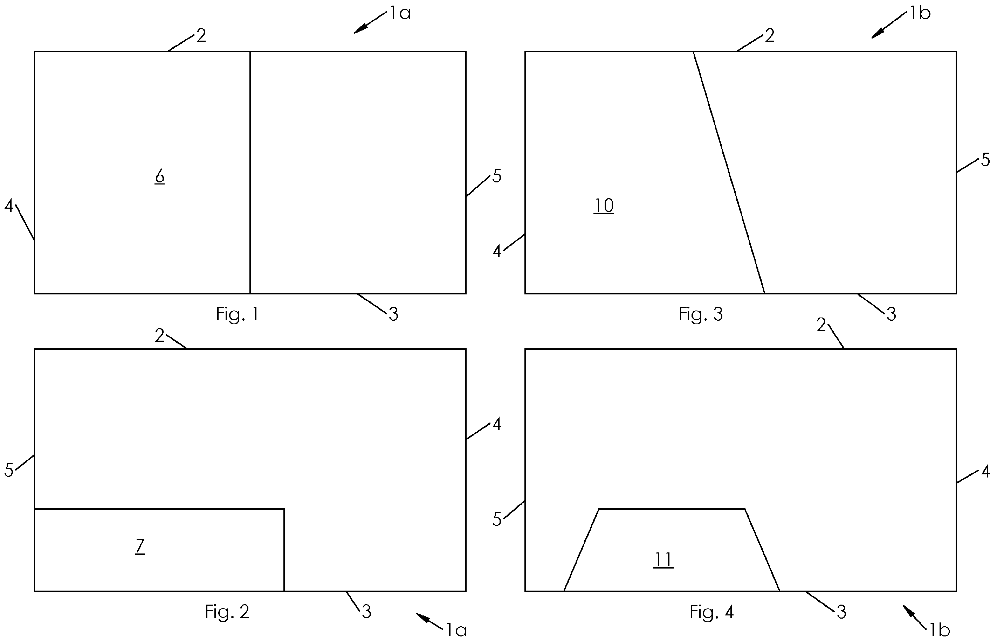

Les prothèses décrites ci-dessous, en lien avec les figures 1 à 6, le sont en référence à la réparation d'une hernie inguinale, et sont destinées à être implantées par voie pré-péritonéale endoscopique ou ouverte. Il est entendu toutefois que les prothèses peuvent trouver une utilisation également avantageuse dans d'autres traitements chirurgicaux, dans lesquels se posent des problèmes analogues à ceux identifiés dans l'état de la technique présenté en introduction.

Les prothèses pour la réparation d'une hernie inguinale sont représentées sur les figures pour une hernie du côté droit. Il est entendu qu'une prothèse analogue, non représentée, peut être employée pour la réparation d'une hernie inguinale du côté gauche, les prothèses droite et gauche étant en symétrie l'une de l'autre.

Les prothèses qui vont être décrites sont de type implant plan. Il est entendu toutefois que l'invention vise également les prothèses dites anatomiques, ou tridimensionnelles, issues d'un thermoformage, ou comprenant des coutures, de sorte à épouser les formes anatomiques de l'espace inguinal.

Les prothèses qui vont être décrites comprennent un textile, notamment un textile ajouré en matériau biocompatible, comprenant une première face, dite face pariétale, destinée à être placée en regard des tissus biologiques de la région inguinale, et une deuxième face, opposée à ladite première face, dite face péritonéale, destinée à être placée en regard du péritoine.

La face péritonéale comprend, sur une partie de sa surface, une première zone pourvue d'un revêtement anti adhérent et la face pariétale comprend, sur une partie de sa surface, une deuxième zone pourvue d'un revêtement anti adhérent.

Les deux zones de revêtement anti-adhérent correspondent à l'appareil vésico-prostatique et à la zone de curage ganglionnaire. La face pariétale de la prothèse est appliquée contre la paroi musculaire. La face opposée, dite péritonéale, est en regard de la face profonde du péritoine et de la face antérieure de la vessie.

Plus précisément, la face pariétale de la prothèse recouvre la zone anatomique de curage ganglionnaire, et la face péritonéale de la prothèse est en partie au contact du péritoine et en partie au contact de l'appareil vésico-prostatique, dans une proportion de un tiers /deux tiers, à moitié moitié.

Les prothèses mises en oeuvre selon la présente invention sont avantageusement inaltérables en milieu biologique, non allergéniques, non carcinogènes, stérilisables, et conduisent à une faible réaction inflammatoire.

Dans certaines mises en oeuvre, la prothèse est un textile, notamment un textile ajouré, formé d'un matériau non résorbable, tel que polyamide, polyester, notamment polyéthylène téréphtalate, polypropylène, polytétrafluroréthylène, polyfluorure de vinylidène.

Dans d'autres mises en oeuvre, la prothèse est un textile, notamment un textile ajouré, formé d'un matériau résorbable, par exemple polyglactine.

Avantageusement, lorsque le tissu formant la prothèse est peu transparent, les zones anatomiques sous-jacentes sont matérialisées, sur la prothèse, par un marquage, par exemple sous forme de lignes. Ce marquage permet de délimiter des zones dans lesquelles, le cas échéant, une fixation par agrafe est possible, et d'indiquer les zones dans lesquelles une fixation par agrafe est à éviter.

Le matériau anti-adhérent se présente, dans une mise en oeuvre, sous la forme d'un film non résorbable, par exemple en polytétrafluroréthylène (PTFE).

Dans d'autres mises en oeuvre, le matériau anti adhérent est à base de collagène, ou de silicone, ou de polyuréthane.

Dans d'autres mises en oeuvre, la prothèse comprend un mélange de matériaux résorbables et non résorbables. Dans des mises en oeuvre particulières, la prothèse comprend un textile, notamment un textile ajouré, en polypropylène ou en un mélange polypropylène - polyéthylène téréphtalate, la première et la deuxième zone non adhérente étant formées d'un revêtement à base d'hydrogel, ce revêtement se présentant sous la forme d'un film continu, ou sous la forme de bandes ou de points.

Avantageusement, le matériau anti-adhérent présente une mouillabilité (ou caractère hydrophile) faible, et est par exemple à base de polytétrafluoroéthylène, ou sa forme étirée après chauffage, dite polytétrafluoroéthylène expansé. Dans d'autres réalisations, le matériau anti adhérent est à base de poly-diméthyle siloxane ou de fluorure de polyvinyle.

Avantageusement, le matériau anti-adhérent est enduit sur le textile, notamment le textile ajouré, de la prothèse. Par exemple, la prothèse comprend une enduction locale d'un matériau à base de collagène.

Dans des mises en oeuvre particulières, la prothèse est formée d'un tissu, notamment un tissu ajouré, en polyester, pourvu d'une première zone et d'une deuxième zone anti adhérentes formées d'un revêtement à base de collagène, ce revêtement se présentant sous la forme d'un film continu, ou sous la forme de bandes ou de points.

Dans d'autres mises en oeuvre particulières, la prothèse est formée d'un tissu, notamment un tissu ajouré, en polypropylène, pourvu d'une première zone et d'une deuxième zone anti adhérentes formées d'un revêtement à base de silicone, ce revêtement se présentant sous la forme d'un film continu, ou sous la forme de bandes ou de points.

Dans d'autres mises en oeuvre particulières, la prothèse est à base de polytétrafluoroéthylène, en particulier polytétrafluoroéthylène expansé, et comprend une première zone et une deuxième zone anti adhérentes lisses, le reste des faces de la prothèse étant strié.

Dans d'autres mises en oeuvre, le matériau anti adhérent comprend un gel résorbable, par exemple à base d'acide hyaluronique, ou d'un mélange d'atelcollagène et de maltodextrine.

Dans une autre mise en oeuvre, le matériau anti-adhérent comprend un film résorbable, par exemple à base de cellulose régénérée et oxydée, ou bien à base d'un mélange d'acide polylactique et de coprolactone, ou bien à base d'un mélange d'acide hyaluronique et de carboxyméthylcellulose, ou bien encore à base d'un mélange de collagène, de polyéthylène glycol et de glycérol.

L'on décrit maintenant des exemples de réalisation de la géométrie des prothèses.

Dans le mode de réalisation des figures 1 à 4, la prothèse 1a, 1b est de forme générale rectangulaire et plane, et comprend un bord 2 longitudinal supérieur, un bord 3 longitudinal inférieur, et deux bords latéraux 4, 5.

Dans le mode de réalisation des figures 1 et 2, la première zone 6 de revêtement anti adhérent, sur la face péritonéale, s'étend sur une surface sensiblement rectangulaire, sur toute la largeur de la prothèse et sur une longueur comprise entre le tiers et les deux tiers de la longueur de la prothèse. Ainsi, à titre d'exemple, lorsque la prothèse est rectangulaire, de longueur 15 cm et de largeur 10 cm, la première zone 6 de revêtement anti adhérent s'étend sur 5 à 8 cm, depuis le bord latéral 4. Cette disposition permet de prévenir l'adhérence de la prothèse 1a à la face antérieure de l'appareil vésico-prostatique. En variante de réalisation, non représentée, la zone anti-adhérente 6 est décalée par rapport au bord latéral 4.

Dans le mode de réalisation des figures 1 et 2, la deuxième zone 7 de revêtement anti adhérent, sur la face pariétale, s'étend sur une surface sensiblement rectangulaire, sur le tiers à la moitié de la largeur de la prothèse 1a et sur sensiblement la moitié de la longueur de la prothèse 1a. La deuxième zone 7 de revêtement anti-adhérent, sur la face pariétale, est située dans l'angle inféro-latéral de la prothèse 1a. Ainsi, à titre d'exemple, lorsque la prothèse est rectangulaire, de longueur 15 cm et de largeur 10 cm, la deuxième zone 7 de revêtement anti adhérent s'étend sur 5 à 8 cm depuis le bord latéral 5, et s'étend sur une largeur de 3.5 cm environ. La zone anti-adhérente peut être décalée par rapport au côté 5, de sorte à laisser une zone adhérente limitée en dehors des vaisseaux.

Dans le mode de réalisation des figures 3 et 4, la première zone 10 de revêtement anti adhérent, sur la face péritonéale, s'étend sur une surface sensiblement trapézoïdale, dont la hauteur est sensiblement égale à la largeur de la prothèse 1b, le grand côté étant situé au voisinage du bord 3 longitudinal inférieur de la prothèse 1b, le petit côté étant situé au voisinage du bord 2 longitudinal supérieur de la prothèse 1b. Ainsi, à titre d'exemple, lorsque la prothèse 1b est rectangulaire, de longueur 15 cm et de largeur 10 cm, le grand côté de la zone 10 de revêtement anti adhérent mesure entre 5 et 8 cm, le petit côté mesurant entre 3 et 7 cm.

Dans le mode de réalisation des figures 3 et 4, la deuxième zone 11 de revêtement anti adhérent, sur la face pariétale, s'étend sur une surface sensiblement trapézoïdale, dont le grand côté s'étend sensiblement sur le bord longitudinal inférieur 3 de la prothèse 1b. A titre d'exemple, la prothèse ayant une longueur de 15 cm et une largeur de 10 cm, la deuxième zone 11 de forme trapézoïdale s'étend sur une hauteur de 3 à 5 cm, son grand côté mesurant 5 à 6 cm, et son petit côté mesurant de 1 à 2 cm de moins que son grand côté. Avantageusement, cette disposition permet de maintenir une zone d'adhérence de la prothèse au plan pariétal d'environ 2 cm entre le bord latéral 4 de la prothèse 1b et la deuxième zone 11. En variante, la zone anti-adhérente trapézoïdale est décalée par rapport au bord 5, de sorte à laisser une zone adhérente un peu plus étendue.

Dans le mode de réalisation des figures 5 et 6, la prothèse 1c est de forme ovale. La première zone 20 de revêtement anti adhérent, sur la face péritonéale, s'étend sur toute la largeur de la prothèse 1c et sur la moitié et jusqu'aux deux tiers de la surface de la prothèse 1c. La deuxième zone 21 de revêtement anti adhérent, sur la face pariétale, s'étend sur sensiblement la moitié de la longueur de la prothèse 1c.

Avantageusement, la prothèse est de type anatomique et comprend une première partie, dite supérieure, destinée à être placée en regard de la paroi musculaire antérieure, et du ligament pectiné, et une deuxième partie, dite partie inférieure, destinée à être placée en regard du pubis en dedans et des vaisseaux iliaques et spermatiques, latéralement, et d'une partie du muscle psoas, la partie supérieure représentant entre la moitié et les deux tiers de la surface de la prothèse. Le textile comprend une ligne délimitant une frontière entre la partie supérieure et la partie inférieure, cette ligne comprenant avantageusement une couture. Cette couture facilite, lors du dépliage de la prothèse, son adaptation aux formes anatomiques de la région inguinale. La partie supérieure de la prothèse sert à la fixation de celle-ci, par des moyens connus en eux-mêmes (par exemple colle, agrafe), cette fixation n'étant pas obligatoire, ainsi qu'il a été dit précédemment, la prothèse étant appliquée contre la paroi par la pression abdominale

Avantageusement, la prothèse est pourvue d'un moyen d'indication de son orientation, par exemple une zone ayant une couleur différente du reste de la prothèse. Le textile étant par exemple sous la forme d'un tricot, la zone de couleur différente est obtenue par tricotage d'un fil de couleur différente du ou des fils utilisés pour le tricotage du reste du textile, ou par impression.

Dans d'autres mises en oeuvre, non représentées, la prothèse est destinée au traitement de l'incontinence urinaire, et est de type bandelette sous urétrale.

Pour le traitement de l'incontinence urinaire, on connait la mise en place de bandelettes, sous la forme d'un treillis réalisé en matériau polymère, ces bandelettes formant hamac soutenant l'urètre. Le même principe est utilisé conventionnellement pour le traitement de l'incontinence associée à la cystocèle, variété de prolapsus génital souvent désignée par l'expression « descente de vessie », ce prolapsus apparaissant par glissement de la paroi vésicale, qui déroule la paroi antérieure du vagin et forme une saillie. Ce traitement est appelé par certains TICT *(*Leanza et al, How to prevent mesh érosion in transobturator tension-free incontinence cystocele treatment TICT: a comparative survey, G. Chir, pp.21-25 , 2015)*.* L'intervention est pratiquée par voie vaginale, avec incision de la paroi antérieure du vagin, pour placer le hamac, et les deux extrémités de la bandelette sont introduites de chaque côté au moyen de trocarts pleins.

Une des complications de ces prothèses est l'ulcération de la paroi vaginale ou de l'urètre, au contact du matériel prothétique avec extériorisation de la prothèse dans le vagin, infection, douleur, saignement, troubles sexuels, et parfois ulcération de la vessie.

L'incidence de ces complications est variable, selon les différentes techniques de placement, l'expérience des chirurgiens *(*Surkont et al, Long term risk of complications after mid-urethral slings IVS implantation Ann Agri Environ Med, 22(1), pp. 163-166, 2015)*.* Cette incidence est de :
- 5% environ dans la série de Leanza et al *(*Leanza et al, How to prevent mesh érosion in transobturator tension-free incontinence cystocele treatment TICT: a comparative survey, G. Chir, pp.21-25, 2015*);*
- 4.3 à 13.8 % dans la série de Boudry et al (Sling exposure after treatment of urinary incontinence with sub-urethral transobturator slings, Eur J Obstet Gynecol Reprod Biol, 176, pp. 191-196, 2014*);*
- 15% dans la série de Viereck et al (Midurethral sling incision: indications and outcomes, Int Urogynecol J, 24, pp. 645-653, 2013*);*
- 11.9 % dans la série de Taner et al (Perioperative and postoperative complications after Ophira mini sling operations, Arch Gynecol Obstet, 291, pp. 341-346, 2015*).*

Sur une série de 111 femmes souffrant de complications de la prothèse, l'extrusion représente, selon Hansen et al, 65% des cas (Long term follow-up of treatment for synthetic mesh complications, Femele Pelvic Med Reconstr Surg, 20, pp. 126-130, 2014*).*

Des cas de cancer de l'intestin ont été observés après sacrocolpopexie, l'irritation chronique par la prothèse étant un facteur contribuant à l'apparition de ces cancers *(*Ahuja et al, Bowel cancer and previous mesh surgery, Gynecol Surg, 8, pp. 217-221, 2011*).*

Comme indiqué dans le document FR 2802798, on connait des dispositifs de traitement de l'incontinence urinaire, se présentant sous forme d'une sangle soutenant l'urètre, ces sangles pouvant provoquer des frottements dans la région du vagin, de l'urètre ou de la vessie, entraînant une érosion, des inflammations, ou des infections, voire provoquer le rejet de la sangle et nécessiter son retrait. Pour tenter de résoudre ces problèmes, le document FR 2802798 propose de fixer, sur une face de la bandelette formant la sangle, un coussinet amortissant en mousse, ou une capsule contenant de l'air, de l'eau, de l'huile ou un gel de silicone, l'épaisseur du coussinet étant de vingt fois celle de la bandelette.

La mise en oeuvre d'une bandelette pourvue d'un tel coussinet ou d'une telle capsule est complexe. En particulier, la présence du coussinet ou de la capsule rend l'introduction par trocart très difficile, voire impossible.

L'enlèvement de bandelette peut nécessiter une chirurgie invasive ou multiple (Braun et al, Mesh removal following transvaginal mesh placement : a case series of 104 operations, Int Urogynecol J, 21, pp. 423-430, 2010), et il est parfois impossible d'enlever complètement cette bandelette, les complications de la mise en place de la prothèse étant alors permanentes, en particulier la présence de douleurs.

Les présents inventeurs proposent, pour réduire les risques associés aux bandelettes de traitement de l'incontinence, de modifier localement la prothèse, en prévoyant une première zone anti-adhérente et une deuxième zone anti-adhérente placées chacune sur une face de la prothèse.

Le textile employé pour la prothèse sous urétrale est avantageusement plus souple qu'un matériau employé pour la réalisation d'une prothèse de réparation de hernie inguinale. Cette disposition permet de préserver la souplesse et la flexibilité du plancher pelvien, permettant une adaptation aux mouvements et aux forces associés à la vie quotidienne, notamment la toux, la défécation, la marche, les relations sexuelles.

Le textile employé présente avantageusement des pores de plus de 2 mm d'ouverture, et une densité faible, de moins de 35g/m².

La prothèse sous urétrale présente avantageusement un revêtement anti adhérent sur sa face en contact avec la paroi vaginale (dite face vaginale) et/ou un revêtement anti-adhérent sur sa face en contact avec l'urètre et/ou la vessie (dite face vésico-urétrale).

Par « *revêtement anti adhérent »* on désigne ici la présence d'un matériau anti adhérent sur la surface du textile de la prothèse, et/ou un état de surface à faible rugosité.

Le revêtement anti adhérent présent dans la première zone anti adhérente peut être identique ou être différent du revêtement anti adhérent présent dans la deuxième zone anti adhérente. Ainsi, par exemple, un revêtement à base de silicone ou d'hydrogel forme la première zone anti adhérente, la deuxième zone anti adhérente étant définie par la présence, dans cette deuxième zone, d'une très faible rugosité de surface.

Selon diverses mises en oeuvre, le revêtement se présente sous la forme d'un film continu, ou sous la forme de bandes ou de points. Par « *points »* on désigne ici une étendue de dimension réduite, ces points pouvant former un motif régulier, ou être disposés aléatoirement.

Dans une mise en oeuvre, la prothèse sous urétrale de traitement de l'incontinence comprend une zone anti-adhérente qui s'étend sur toute la largeur de la bandelette et mesure, par exemple, entre 3 et 4 cm de long sur la face vaginale, et entre 1cm et 2.5 cm sur la face vésico-urétrale.

Dans une mise en oeuvre, la prothèse de traitement de la cystocèle comprend une partie centrale et des bras, la partie centrale comprenant une zone anti-adhérente qui couvre plus de 80% de la surface de cette partie centrale, sur l'une de ses faces et laisse libre un bord périphérique de cette partie centrale, sur une largeur de quelques millimètres, par exemple de 5mm à 15 mm.

Dans d'autres mises en oeuvre, la prothèse est adaptée au traitement du prolapsus génital et est destinée à être placée au contact de la paroi vaginale postérieure, la prothèse comprenant, sur la face au contact de la paroi vaginale, au moins une zone anti-adhérente continue, ou discontinue, par exemple sous forme de bandes ou de points.

## Revendications

1. Prothèse (**1a**, **1b, 1c**) pour la réparation d'une hernie inguinale, destinée à être implantée par voie pré-péritonéale endoscopique ou ouverte, comprenant un textile ajouré en matériau biocompatible, comprenant une première face, dite face pariétale, destinée à être placée en regard des tissus biologiques de la région inguinale, et une deuxième face, opposée à ladite première face, dite face péritonéale, destinée à être placée en regard du péritoine et de la face antérieure de la vessie, **caractérisée en ce que** la face péritonéale comprend, sur une partie de sa surface, une première zone **(6, 10, 20)** pourvue d'un revêtement anti adhérent, la face pariétale comprenant, sur une partie de sa surface, une deuxième zone **(7, 11 21)** pourvue d'un revêtement anti adhérent, la prothèse étant de forme rectangulaire, comprenant un bord (2) longitudinal supérieur, un bord (3) longitudinal inférieur, et deux bords latéraux (4, 5), la première zone s'étendant sur toute la largeur de la prothèse depuis un bord latéral (4) de la prothèse, la deuxième zone s'étendant sur une largeur comprise entre le tiers et la moitié de la largeur de la prothèse, la première zone et la deuxième zone n'étant pas disposées sensiblement à la verticale l'une de l'autre lorsque la prothèse est vue en plan.

2. Prothèse selon la revendication 1, **caractérisée en ce que** la première zone **(6, 10, 20)** s'étend sur une surface de l'ordre de 30% à 70% de la surface totale de la face péritonéale.

3. Prothèse selon l'une quelconque des revendications précédentes, **caractérisée en ce que** la première zone **(6, 10, 20)** et/ou la deuxième zone **(7, 11, 21)** comprend un revêtement anti adhérent définissant un film continu.

4. Prothèse selon l'une quelconque des revendications 1 à 3 **caractérisée en ce que** le revêtement anti adhérent de la première zone **(6, 10, 20)** et/ou de la deuxième zone **(7, 11, 21)** est discontinu et comprend des bandes ou points.

5. Prothèse selon l'une quelconque des revendications précédentes, **caractérisée en ce que** le textile ajouré est réalisé à partir de polyéthylène, polypropylène, polyéthylène téréphtalate, polytétrafluoroéthylène, polyflurorure de vinylidène.

6. Prothèse selon l'une quelconque des revendications précédentes, **caractérisée en ce que** la première zone **(6, 10, 20)** et/ou de la deuxième zone **(7, 11, 21)** comprend un film non résorbable.

7. Prothèse selon l'une quelconque des revendications 1 à 6 **caractérisée en ce que** la première zone **(6, 10, 20)** et/ou de la deuxième zone **(7, 11, 21)** comprend un gel résorbable.

8. Prothèse selon l'une quelconque des revendications précédentes, **caractérisée en ce que** le textile comporte une première partie, dite partie supérieure, destinée à être placée en regard de la paroi musculaire antérieure, et du ligament pectiné, et une deuxième partie, dite partie inférieure, destinée à être placée en regard du pubis et des vaisseaux iliaques et spermatiques, latéralement, et d'une partie du muscle psoas, la partie supérieure représentant environ entre la moitié et les deux tiers de la surface de la prothèse, le textile comprenant une ligne délimitant une frontière entre la partie supérieure et la partie inférieure.

9. Prothèse selon la revendication 8, **caractérisée en ce que** la ligne délimitant la frontière entre la partie supérieure et la partie inférieure de la prothèse comprend une couture.

10. Prothèse selon l'une quelconque des revendications précédentes, **caractérisée en ce qu'**elle est pourvue d'un moyen d'indication de l'orientation de la prothèse.

11. Prothèse selon la revendication précédente, **caractérisée en ce que** ledit moyen d'indication de l'orientation de la prothèse comprend une zone ayant une couleur différente du reste de la prothèse.

12. Prothèse selon la revendication précédente, **caractérisée en ce que,** le textile étant sous la forme d'un tricot, la zone de couleur différente est obtenue par tricotage d'un fil de couleur différente du ou des fils utilisés pour le tricotage du reste du textile, ou par impression.

## Patentansprüche

1. Prothese (**1a, 1b, 1c**) zur Reparatur einer Leistenhernie, die zur endoskopischen oder offenen präperitonealen Implantation bestimmt ist, umfassend ein durchbrochenes Textil aus einem biokompatiblen Material, umfassend eine erste Seite, bezeichnet als Parietalseite, die bestimmt ist, dem biologischen Gewebe des Leistenbereichs zugewandt platziert zu sein, und eine zweite Seite, bezeichnet als Peritonealseite, die der ersten Seite gegenüberliegt, die bestimmt ist, dem Peritoneum und der vorderen Seite der Blase zugewandt platziert zu sein, **dadurch gekennzeichnet, dass** die Peritonealseite auf einem Teil ihrer Oberfläche einen ersten Bereich **(6, 10, 20)** umfasst, der mit einer Antihaftbeschichtung versehen ist, wobei die Parietalseite auf einem Teil ihrer Oberfläche einen zweiten Bereich **(7, 11, 21)** umfasst, der mit einer Antihaftbeschichtung versehen ist, wobei die Prothese rechteckig ist, umfassend einen oberen Längsrand (2), einen unteren Längsrand (3) und zwei Seitenränder (4, 5), wobei sich der erste Bereich über die gesamte Breite der Prothese ab einem Seitenrand (4) der Prothese erstreckt, wobei sich der zweite Bereich über eine Breite erstreckt, die zwischen dem Drittel und der Hälfte der Breite der Prothese liegt, wobei der erste Bereich und der zweite Bereich nicht im Wesentlichen senkrecht zueinander angeordnet sind, wenn die Prothese in der Ebene betrachtet wird.

2. Prothese nach Anspruch 1, **dadurch gekennzeichnet, dass** sich der erste Bereich **(6, 10, 20)** über eine Oberfläche in der Größenordnung von 30 % bis 70 % der Gesamtfläche der Peritonealseite erstreckt.

3. Prothese nach einem der vorhergehenden Ansprüche, **dadurch gekennzeichnet, dass** der erste Bereich **(6, 10, 20)** und/oder der zweite Bereich **(7, 11, 21)** eine Antihaftbeschichtung umfasst, die einen durchgehenden Film definiert.

4. Prothese nach einem der Ansprüche 1 bis 3, **dadurch gekennzeichnet, dass** die Antihaftbeschichtung des ersten Bereichs **(6, 10, 20)** und/oder des zweiten Bereichs **(7, 11, 21)** unterbrochen ist und Streifen oder Punkte umfasst.

5. Prothese nach einem der vorhergehenden Ansprüche, **dadurch gekennzeichnet, dass** das durchbrochene Textil aus Polyethylen, Polypropylen, Polyethylenterephthalat, Polytetrafluorethylen, Polyvinylidenfluorid hergestellt ist.

6. Prothese nach einem der vorhergehenden Ansprüche, **dadurch gekennzeichnet, dass** der erste Bereich **(6, 10, 20)** und/oder der zweite Bereich **(7, 11, 21)** einen nicht resorbierbaren Film umfasst.

7. Prothese nach einem der Ansprüche 1 bis 6, **dadurch gekennzeichnet, dass** der erste Bereich **(6, 10, 20)** und/oder der zweite Bereich **(7, 11, 21)** ein resorbierbares Gel umfasst.

8. Prothese nach einem der vorhergehenden Ansprüche, **dadurch gekennzeichnet, dass** das Textil einen ersten Teil, bezeichnet als oberen Teil, aufweist, der bestimmt ist, der vorderen Muskelwand und dem pektinierten Ligament zugewandt platziert zu sein, und einen zweiten Teil, bezeichnet als unteren Teil, der bestimmt ist, dem Schambein und den iliakalen und Samengefäßen, seitlich, und einen Teil des Psoas-Muskels zugewandt platziert zu sein, wobei der obere Teil etwa zwischen der Hälfte und den zwei Dritteln der Oberfläche der Prothese darstellt, wobei das Textil eine Linie umfasst, die eine Grenze zwischen dem oberen und dem unteren Teil begrenzt.

9. Prothese nach Anspruch 8, **dadurch gekennzeichnet, dass** die Linie, die die Grenze zwischen dem oberen Teil und dem unteren Teil der Prothese begrenzt, eine Naht umfasst.

10. Prothese nach einem der vorhergehenden Ansprüche, **dadurch gekennzeichnet, dass** sie mit einem Mittel zur Anzeige der Ausrichtung der Prothese versehen ist.

11. Prothese nach vorhergehendem Anspruch, **dadurch gekennzeichnet, dass** das Mittel zur Anzeige der Ausrichtung der Prothese einen Bereich mit einer anderen Farbe als der Rest der Prothese umfasst.

12. Prothese nach vorhergehendem Anspruch, **dadurch gekennzeichnet, dass** das Textil in Form eines Gewirks vorliegt, der unterschiedlich farbige Bereich durch Wirken eines Garns mit einer anderen Farbe als das oder die Garne, die zum Wirken des Rests des Textils verwendet werden, oder durch Druck erhalten wird.

## Claims

1. A prosthesis (**1a, 1b**, **1c** ) for inguinal hernia repair, for being implanted by endoscopic or open preperitoneal pathway, comprising an openwork textile of a biocompatible material, comprising a first so-called parietal face, for being placed facing the biological tissues of the inguinal region, and a second so-called peritoneal face, opposite to said first face, for being placed facing the peritoneum and the anterior face of the bladder, **characterised in that** the peritoneal face comprises, on part of its surface, a first zone (**6, 10, 20**) provided with an anti-adherent coating, the parietal face comprising, on part of its surface, a second zone **(7,11 21)** provided with a non-adherent coating, the prosthesis being rectangular in shape, comprising an upper longitudinal edge (2), a lower longitudinal edge (3), and two side edges (4, 5), the first zone extending over the entire width of the prosthesis from a side edge (4) of the prosthesis, the second zone extending over a width of between one-third and one-half of the width of the prosthesis, the first zone and the second zone not being arranged substantially vertically to one another when the prosthesis is seen in a plane view.

2. The prosthesis according to claim 1, **characterised in that** the first zone **(6, 10, 20)** extends over an area in the order of 30% to 70% of the total area of the peritoneal face.

3. The prosthesis according to any one of the preceding claims, **characterised in that** the first zone **(6, 10, 20)** and/or the second zone (**7, 11, 21**) comprises an anti-adhesion coating defining a continuous film.

4. The prosthesis according to any one of claims 1 to 3, **characterised in that** the non-adherent coating of the first zone **(6, 10, 20)** and/or of the second zone **(7, 11, 21)** is discontinuous and comprises strips or dots.

5. The prosthesis according to any one of the preceding claims, **characterised in that** the openwork textile is made from polyethylene, polypropylene, polyethylene terephthalate, polytetrafluoroethylene, polyvinylidene fluoride.

6. The prosthesis according to any one of the preceding claims, **characterised in that** the first zone **(6, 10, 20)** and/or the second zone **(7, 11, 21)** comprises a non-resorbable film.

7. The prosthesis according to any one of claims 1 to 6, **characterised in that** the first zone **(6, 10, 20)** and/or the second zone **(7, 11, 21)** comprises a resorbable gel.

8. The prosthesis according to any one of the preceding claims, **characterised in that** the textile comprises a first so-called upper part, for being placed facing the anterior muscle wall, and the pectinate ligament, and a second so-called lower part, for being placed facing the pubis and iliac and spermatic vessels, laterally, and part of the psoas muscle, the upper part accounting for about half to two thirds of the area of the prosthesis, the textile comprising a line delimiting a boundary between the upper part and the lower part.

9. The prosthesis according to claim 8, **characterised in that** the line delimiting the boundary between the upper part and the lower part of the prosthesis comprises a seam.

10. The prosthesis according to any one of the preceding claims, **characterised in that** it is provided with a means for indicating orientation of the prosthesis.

11. The prosthesis according to the preceding claim, **characterised in that said** means for indicating orientation of the prosthesis comprises a zone having a colour different from the rest of the prosthesis.

12. The prosthesis according to the preceding claim, **characterised in that**, the textile being in the form of a knit, the different-colour zone is obtained by knitting a yarn of a different colour from the yarn(s) used for knitting the rest of the textile, or by printing.
